Europäisches Patentamt

European Patent Office

Office européen des brevets

(19)

(11) Veröffentlichungsnummer: **0 291 819 B1**

# (12) EUROPÄISCHE PATENTSCHRIFT

(45) Veröffentlichungstag der Patentschrift: **08.07.92**

(21) Anmeldenummer: **88107468.6**

(22) Anmeldetag: **10.05.88**

(51) Int. Cl.5: **C07C 263/10**, B01F 7/00, B01F 5/06, B01F 5/22

(54) **Verfahren zur Herstellung von Isocyanaten.**

(30) Priorität: **21.05.87 DE 3717057**

(43) Veröffentlichungstag der Anmeldung:
**23.11.88 Patentblatt 88/47**

(45) Bekanntmachung des Hinweises auf die
Patenterteilung:
**08.07.92 Patentblatt 92/28**

(84) Benannte Vertragsstaaten:
**BE DE ES FR GB IT NL**

(56) Entgegenhaltungen:
DE-A- 2 363 888
DE-A- 2 740 789
DE-A- 2 950 216
DE-C- 2 153 268
GB-A- 2 169 814

(73) Patentinhaber: **BAYER AG**

**W-5090 Leverkusen 1 Bayerwerk(DE)**

(72) Erfinder: **Sauer, Heinz**
**Hubert-Drecker-Strasse 14**
**W-5068 Odenthal-Hahnenberg(DE)**
Erfinder: **Dallmeyer, Hermann, Dr.**
**Im Alten Driesch 22**
**W-5068 Odenthal(DE)**
Erfinder: **Zarnack, Uwe-Jens, Dr.**
**Ulitzhörn 2**
**W-2212 Brunsbüttel(DE)**
Erfinder: **Keggenhoff, Berthold, Dr.**
**Im Paradies 32**
**W-4150 Krefeld 29(DE)**
Erfinder: **Weber, Bernd, Dipl.-Ing.**
**Gertrudissstrasse 6**
**W-4150 Krefeld 1(DE)**

EP 0 291 819 B1

**Beschreibung**

Die Erfindung betrifft ein Verfahren zum Herstellen von Isocyanaten durch

a) Vermischen und Umsetzen von Lösungen bzw. Suspensionen primärer Amine bzw. deren Salze mit Phosgenlösung, wobei die beiden Stoffe zum Einleiten der Umsetzung kontinuierlich in eine Mischzone, welche mindestens eine Rotorscheibe aufweist, eingebracht werden und das entstandene Vorprodukt wieder ausgeführt wird,

b) weitere Umsetzung der entstandenen Primärprodukte unter Erhitzen,

c) Aufarbeitung der erhaltenen Isocyanatlösung zu den entsprechenden Roh- und/oder Reinisocyanaten.

Isocyanate werden in der chemischen Technik überwiegend durch Umsetzung von primären Aminen mit Phosgen hergestellt, wobei beide Reaktionskomponenten üblicherweise in einem geeigneten Lösungsmittel gelöst sind.

Die Vermischungsgüte dieser beiden Reaktionskomponenten hat erheblichen Einfluß auf den Reinheitsgrad des erzeugten Vorproduktes, wobei es auch von der Art des Mischers abhängt, ob das Produkt ohne Klumpenbildung und ohne Anbackungen entsteht.

Man hat sich hierzu spezieller Düsenanordnungen bedient (DE-PS 17 92 660 entsprechend GB-PS 12 38 669; DE-OS 29 50 216 entsprechend US-PS 42 89 732; EP-OS 65 727 entsprechend US-PS 44 19 295) oder pumpenähnlicher Aggregate, wie beispielsweise Kreiselpumpen (DE-PS 21 53 268 entsprechend US-PS 37 13 833). Trotz aller Bemühungen lassen sich bei der Bermischung der beiden Stoffe zu einem Vorprodukt Klumpenbildung und Verstopfungen nicht vermeiden.

Es besteht die Aufgabe, das Verfahren der eingangs genannten Art dahingehend zu verbessern, daß Klumpenbildungen und Anbackungen während der ersten Verfahrensstufe vermieden werden.

Dies wird dadurch erreicht, daß bei Durchführung des Schrittes a) die Phosgenlösung axial zur Rotorscheibe zugeführt wird und das in Lösung oder Suspension befindliche Amin parallel zum, aber mit Abstand vom Strom der Phosgenlösung gegen die Rotorscheibe verdüst wird.

Dadurch, daß die Phosgenlösung zentral gegen die Rotorscheibe geführt wird und an dieser radial nach außen abströmen muß und dabei mit der eingedüsten Aminlösung bzw. -suspension beaufschlagt wird, entsteht durch die Rotationskomponente eine sehr gute Vermischung bei engem Verweilzeitspektrum. Aufgrund dessen ist die Gefahr von Klumpenbildungen und Anbackungen minimiert. Durch entsprechende Gestaltung der Rotorscheiben und Statorscheiben hinsichtlich Stärke, Abstand voneinander sowie Durchströmspalt zum Gehäuse bzw. zur Welle kann der Fachmann Einfluß auf die Strömungsverhältnisse nehmen und dadurch insbesondere die Rückvermischung gering halten.

Vorzugsweise wird das in Lösung oder in Suspension befindliche Amin an mehreren Stellen, die den Strom der Phosgenlösung kranzartig umgeben, eingedüst. Es versteht sich, daß man dabei die Düsen am besten in gleichen Winkelabständen anordnet, weil dadurch die besten Voraussetzungen für eine gleichmäßige Vermischung gegeben sind.

Gemäß einer weiteren besonderen Durchführungsform des neuen Verfahrens werden beide Stoffe unter fortschreitender Vermischung und Umsetzung durch mehrere Statorscheiben-Rotorscheiben-Einheiten geführt.

Diese Variante ist insbesondere dann anzuwenden, wenn hohe Durchsatzleistungen verlangt werden, damit eine intensive Vorphosgenierung stattfindet. Insbesondere kann die Stirnwand des Mischers, durch welche die Stoffe zugeführt werden, gleichzeitig als erste Statorscheibe verwendet werden.

Zur Verbesserung der Vermischung versieht man die Rotorscheiben und/oder Statorscheiben vorzugsweise mit Rührelementen, wie beispielsweise aufgesetzten Stiften, konzentrischen Ringen mit Durchbrüchen oder man versieht die Scheiben mit durchbrüchen oder Ausschnitten. Im Sinne der Erfindung ist jeder symmetrische Körper eingeschlossen, der im Prinzip aus einer Scheibe durch Ausschnitte und/oder Aufsätze herstellbar ist.

Für ein enges Verweilzeitspektrum kann zusätzlich auch dadurch gesorgt werden, daß das Vorprodukt mittels mindestens eines Laufrades abgefördert wird. Solche Laufräder sind in der Regel auf der gleichen Welle wie die Rotorscheiben angeordnet.

Der Rotor wird mit einer Drehzahl zwischen 200 und 10 000 min$^{-1}$, vorzugsweise zwischen 1000 und 8000 min$^{-1}$ angetrieben.

Da das eingesetzte Phosgen toxisch ist, muß die gesamte Anlage hermetisch abgeschlossen sein. Für den Antrieb der Welle sieht man deshalb vorzugsweise eine mit einem Spalttopf oder einem Spaltrohr versehene Magnetkupplung vor.

Das neue Verfahren eignet sich zur Phosgenierung beliebiger primärer Mono- und Polyamine, insbesondere zur Herstellung der in der Polyurethanchemie üblichen Polyisocyanate. Ausgangsmaterialien für das erfindungsgemäße Verfahren sind somit neben 3- bis 95 gew.-%igen, vorzugsweise 20- bis 75 gew.-%igen Phosgenlösungen in geeignete Lösungsmitteln, 5- bis 95-gew.-%ige, vorzugsweise 5- bis 50-gew.-%ige Lösungen von Mono- oder Polyaminen in geeigneten Lösungsmitteln. Für das neue Verfahren geeignete Amine sind beliebige primäre Mono- oder Polyamine wie z.B. Methylamin, Ethylamin, Butylamin, Stearylamin, Phenylamin, p-Tolylamin, 1,4-Diaminobutan, 1,6-Diaminohexan, 1,8-Diaminooctan, 1,4-Diaminobenzol, 2,4-Diaminotoluol, 2,6-Diaminotoluol, Gemische der beiden letztgenannten Isomeren, 2,2′-Diaminodiphenylmethan, 2,4′-Diaminodiphenylmethan, 4,4′-Diaminodiphenylmethan, Gemische der drei letztgenannten Isomeren, Alkyl-substituierte Diamine der Diphenylmethanreihe wie z.B. 3,4′-Diamino-4-methyldiphenylmethan, Polyamingemische der Diphenylmethanreihe wie sie in an sich bekannter Weise durch Anilin/Formaldehyd-Kondensation erhalten werden, p-Xylylendiamin, perhydriertes 2,4- und/oder 2,6-Diaminotoluol, 2,2′-, 2,4′- und/oder 4,4′- Diaminodicyclohexylmethan, 1-Amino-3,3,5-trimethyl-5- aminomethyl-cyclohexan (Isophorondiamin, abgekürzt IPDA), Lysin-ethylester, Lysinaminoethylester, 1,6,11-Triamino-undecan oder 1,5-Naphthylendiamin.

Geeignete Lösungsmittel zur Herstellung der Phosgen- und Aminlösungen sind beliebige, unter den Reaktionsbedingungen inerte Lösungsmittel wie z.B. Chlorbenzol, o-Dichlorbenzol, Toluol, Xylol, Methylenchlorid, Perchlorethylen, Trichlorfluormethan oder Butylacetat. Vorzugsweise werden Chlorbenzol oder o-Dichlorbenzol eingesetzt. Beliebige Gemische der beispielhaft genannten Lösungsmittel können selbstverständlich auch eingesetzt werden. Zweckmäßigerweise wird man für die Aminkomponente und das Phosgen das gleich Lösungsmitel bzw. Lösungsmittelgemisch einsetzen, obwohl dies nicht unbedingt erforderlich ist.

Die Konzentrationen und Mengenverhältnisse von Amin- und Phosgenlösung können in weiten Grenzen variiert werden mit der Maßgabe, daß die Phosgenmenge auf jeden Fall für die Reaktion ausreichend sein muß. Deshalb muß die Phosgenmenge so bemessen sein, daß pro Mol primäre Aminogruppen mindestens 1 Mol Phosgen vorhanden ist. Zweckmäßig wird ein Molverhältnis Phosgen : primäre Aminogruppe von 2:1 bis 30:1, vorzugsweise 2:1 bis 5:1, eingehalten. Die Temperatur der Phosgenlösung kann zweckmäßigerweise etwa -25 bis +25°C, die der Basenlösung etwa +20 bis +100°C betragen. Die Vorphosgenierungsprodukte können durch den Mischer selbst in eine nachgeschaltete Heißphosgenierstufe gefördert werden, wo, gegebenenfalls unter Zuführung von weiterm Phosgen, die Umsetzung zu dem gewünschten Isocyanat durchgeführt wird. Wegen der sehr feinen Verteilung der Vorphosgenierungsprodukte geschieht dies in einer kürzeren Zeit und mit weniger Nebenreaktionen als bei den bisher üblichen Verfahren.

Die dem neuen Vorphosgenierverfahren nachgeschaltete Heißphosgenierstufe kann beliebigem Stand der Technik entsprechen und ist für die Durchführbarkeit des neuen Vorphosgenierverfahrens nicht kritisch. Die Heißphosgenierung kann mit oder ohne Zugabe von zusätzlichem Phosgen unter erhöhtem Druck, leichtem Überdruck oder Normaldruck kontinuierlich oder diskontinuierlich vorgenommen werden.

In einer bevorzugten Ausführung des Verfahrens wird die Heißphosgenierung möglichst kontinuierlich in Türmen unter normalem Druck oder ganz leichtem Überdruck bis etwa 1,5 atü durchgeführt werden. Besonders vorteilhaft ist es, das die Mischvorrichtung verlassende Vorphosgeniergemisch kontinuierlich von oben oder unten in einem beheizbaren Turm eintreten zu lassen und die Reaktion durch Zuführung von Wärme zu vollenden. Man kann auch mehrere Türme hintereinanderschalten oder Kombinationen von Kesseln und Türmen verwenden, wenn zur Optimierung der Reaktionsführung in der Heißphosgenierstufe ein bestimmtes Temperaturprofil eingehalten werden soll.

Die so erhaltene Isocyanatlösung kann in dem Stand der Technik bekannter Weise durch Destillation und/oder Kristallisation zu den Isocyanaten aufgearbeitet werden. Durch das neue Verfahren wird ein besonders niedriger Gehalt an unerwünschten Nebenprodukten und demzufolge bei der Gewinnung der reinen Isocyanate eine besonders hohe Ausbeute erzielt. Bei den Rohisocyanatgemischen ist der niedrige Gehalt an Nebenprodukten an dem hohen Gehalt an freien Isocyanatgruppen erkennbar.

In der Zeichnung ist rein schematisch im Schnitt in zwei Ausführungsbeispielen ein Mischer dargestellt, welcher für die Durchführung der ersten Verfahrensstufe geeignet ist. Es zeigen:

Fig. 1 einen Mischer mit Rührelementen auf den Statorscheiben und auf den Rotorscheiben und

Fig. 2 einen Mischer mit zwei Laufrädern.

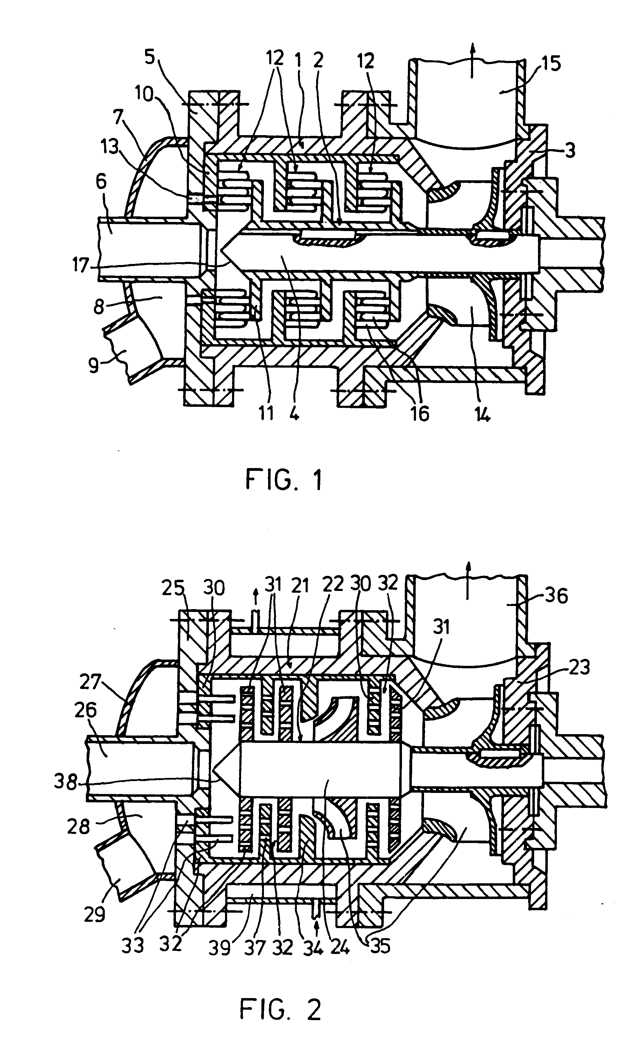

In Fig. 1 besteht der Mischer aus einem Gehäuse 1 und einem Rotor 2. In einer Stirnwand 3 ist die Welle 4 des Rotors 2 geführt und nach außen hermetisch abgedichtet. Der Antrieb erfolgt über eine nicht dargestellte Magnetkupplung. Die andere Stirnwand 5 ist axial zur Welle 4 mit einem Zuführstutzen bzw. Einlaß 6 für die Zuführung einer Phosgenlösung vorgesehen. Der Ztirnwand 5 vorgeordnet ist eine Haube 7, so daß eine Kammer 8 gebildet ist. In sie mündet eine Zuführung 9 für eine Aminlösung. An der Innenseite der Stirnwand 5 ist eine Statorscheibe 10 angeordnet, welche zusammen mit einer ersten, auf der Welle 4 angeordneten Rotorscheibe 11 eine erste Einheit 12 bildet. Von der Kammer 8 ragen Düsen 13 durch die Stirnwand 5 und die Statorscheibe 10 hindurch und münden direkt vor der Rotorscheibe 11. Das Gehäuse 1 umschließt noch zwei weitere, gleichartig aufgebaute Statorscheiben-Rotorscheiben-Einheiten 12, welche in Reihe angeordnet sind. Hinter der letzten Einheit 12 verengt sich das Gehäuse bis zu einem Laufrad 14; in dessen Bereich ist ein Abführstutzen 15 vorgesehen, von welchem eine nicht dargestellte Zuleitung zur nachfolgenden Heißphosgenierstufe führt. Sowohl die Statorscheiben 10 als auch die Rotorscheiben 11 sind mit Stiften als Rührelemente 16 versehen, welche in Form von konzentrischen, ineinandergreifenden Kränzen angeordnet sind. Die Stirnfläche 17 der Welle 4 ist zum Einlaß 6 hin zugespitzt.

In Fig. 2 besteht der Mischer aus einem Gehäuse 21 und einem Rotor 22. In einer Stirnwand 23 ist die Welle 24 des Rotors 22 geführt und nach außen hermetisch abgedichtet. Der Antrieb erfolgt über eine nicht dargestellte Magnetkupplung. Die andere Stirnwand 25 ist axial zur Welle 24 mit einem Zuführstutzen bzw. Einlaß 26 für eine Phosgenlösung versehen. Der Stirnwand 25 vorgeordnet ist eine Haube 27, welche eine Kammer 28 bildet. In diese mündet eine Zuführung 29 für eine Aminsuspension. An der Innenseite der Stirnwand 25 ist eine Statorscheibe 30 angeordnet, welche mit einer auf der Welle 24 angeordneten ersten Rotorscheibe 31 eine Einheit 32 bildet. Durch die Stirnwand 25 und die Statorscheibe 30 ragen aus der Kammer 28 gespeiste Düsen 33 und münden direkt vor der ersten Rotorscheibe 31. Es sind noch zwei weitere Statorscheiben-Rotorscheiben-Einheiten 32 vorgesehen, wobei zwischen der zweiten und dritten Einheit 32 eine zusätzliche Statorscheibe 34 sowie ein erstes Laufrad 35 vorgesehen sind. Hinter der dritten Einheit 32 schnürt sich das Gehäuse 21 bis zu einem Laufrad 35 ein, aus dessen Bereich ein Abführstutzen 36 von Gehäuse 21 abführt. Es schließt sich eine nicht dargestellte, zu einer nachgeordneten Heißphosgenierstufe führende Verbindungsleitung an. Die erste Statorscheibe 30 dient zur Aufnahme der Düsen 33, während die weiteren Statorscheiben 30 und Rotorscheiben 31 Schlitze als Rührelemente 37 aufweisen. Die zum Einlaß 26 gerichtete Stirnfläche 38 der Welle 24 ist zugespitzt. Das Gehäuse 21 ist mit einem Temperiermantel 39 versehen.

## Patentansprüche

1. Verfahren zum Herstellen von Isocyanaten durch
   a) Vermischen und Umsetzen von Lösungen bzw. Suspensionen primärer Amine bzw. deren Salze mit Phosgenlösung, wobei die beiden Stoffe zum Einleiten der Umsetzung kontinuierlich in eine Mischzone, welche mindestens eine Rotorscheibe (11, 31) aufweist, eingebracht werden und das entstandene Vorprodukt wieder ausgeführt wird,
   b) weitere Umsetzung der entstandenen Primärprodukte unter Erhitzen,
   c) Aufarbeitung der erhaltenen Isocyanatlösung zu den entsprechenden Roh- und/oder Reinisocyanaten,
   dadurch gekennzeichnet, daß bei der Durchführung des Schrittes a) die Phosgenlösung axial zur Rotorscheibe (11, 31) zugefügt wird und das in Lösung oder Suspension befindliche Amin parallel zum aber mit Abstand vom Strom der Phosgenlösung gegen die Rotorscheibe (11, 31) verdüst wird.

2. Verfahren nach Anspruch 1, dadurch gekennzeichnet, daß das in Lösung oder in Suspension befindliche Amin an mehreren Stellen (13, 33), die den Strom der Phosgenlösung kranzartig umgeben, eingedüst wird.

3. Verfahren nach Anspruch 1 oder 2, dadurch gekennzeichnet, daß beide Stoffe unter fortschreitender Vermischung und Umsetzung durch mehrere Statorscheiben-Rotorscheiben-Einheiten (12, 32) geführt werden.

4. Verfahren nach einem der Ansprüche 1 bis 3, dadurch gekennzeichnet, daß das entstandene Vorprodukt mittels mindestens eines Laufrades (14, 35) abgeführt wird.

## Claims

1. A process for the production of isocyanates by
   (a) mixing and reacting solutions or suspensions of primary amines or their salts with phosgene solution, during which to start the reaction the two substances are introduced continuously to a mixing zone, which contains at least one rotor disc (11, 31), and the initial product formed is led out again,
   (b) further reaction, by heating, of the primary product formed,
   (c) processing of the isocyanate solution obtained to the corresponding crude and/or pure isocyanates,
   characterized in that during the execution of step (a) the phosgene solution is fed axially to the rotor disc (11, 31) and the amine present in solution or suspension is sprayed parallel to but at a distance from the stream of phosgene solution against the rotor disc (11, 31).

2. A process according to Claim 1, characterized in that the amine present in solution or in suspension is sprayed in at several points (13, 33) which surround the stream of phosgene solution annularly.

3.  A process according to Claim 1 or 2, characterized in that the two substances are led with progressive mixing and reaction through several stator disc - rotor disc units (12, 32).

4.  A process according to one of Claims 1 to 3, characterized in that the initial product formed is drawn off by means of at least one impeller (14, 35).

**Revendications**

1.  Procédé de préparation d'isocyanates par
    a) mélange et réaction de solutions ou de suspensions d'amines primaires ou de leurs sels avec une solution de phosgène, où on introduit les deux produits de façon continue, pour provoquer la réaction, dans une zone de mélange qui présente au moins un disque de rotor (11,31) et où on fait ressortir le produit intermédiaire formé,
    b) transformation ultérieure, par chauffage, des produits primaires obtenus,
    c) traitement final de la solution d'isocyanate obtenue pour donner les isocyanates bruts et/ou purs correspondants,
    caractérisé en ce que, lors de la mise en oeuvre de l'étape a), la solution de phosgène est ajoutée de façon axiale au disque de rotor (11,31) et l'amine se trouvant en solution ou en suspension est vaporisée parallèlement au courant de la solution de phosgène mais à distance de celui-ci, contre le disque de rotor (11,31).

2.  Procédé selon la revendication 1, caractérisé en ce que l'amine se trouvant en solution ou en suspension est injectée en plusieurs endroits (13,33) qui entourent en couronne le courant de la solution de phosgène.

3.  Procédé selon la revendication 1 ou 2, caractérisé en ce que les deux produits sont conduits à travers plusieurs unités disque de stator-disque de rotor (12,32) en subissant un mélange et une réaction progressifs.

4.  Procédé selon l'une des revendications 1 à 3, caractérisé en ce que le produit intermédiaire obtenu est évacué au moyen d'au moins une roue mobile (14,35).

FIG. 1

FIG. 2